# EUROPEAN PATENT APPLICATION

(11) **EP 4 253 364 A1**
(43) Date of publication of application: **04.10.2023**
(21) Application number: 21898402.9
(22) Date of filing: 04.11.2021
(51) Int. Cl.: C07C 67/48, C07C 67/343, C07C 69/708, B01J 23/04

(54) **METHOD FOR PREPARING HETEROLOGOUS LINEAR CARBONATE, WITH CATALYST FILTERING STEP INTRODUCED THEREINTO**

(30) Priority: 26.11.2020 KR 20200161739
(71) Applicant: Lotte Chemical Corporation, Seoul, 05551 (KR)
(72) Inventor: KIM, Wang Gyu, Daejeon 34110 (KR); BAEK, Mi Hwa, Daejeon 34110 (KR); CHOI, Jong Myung, Daejeon 34110 (KR); HAN, Eun Hye, Daejeon 34110 (KR); KIM, Jin Hyung, Daejeon 34110 (KR)
(74) Representative: Germain Maureau
(86) International application number: PCT/KR2021/015844
(87) International publication number: WO 2022/114576

(57) **Abstract**

Provided herein is a method of preparing a heterogeneous linear carbonate, including: performing a transesterification reaction of dimethyl carbonate (DMC) and ethanol (EtOH) in the presence of a catalyst; and filtering a composite obtained by the transesterification reaction through a filter.

## Description

### [Technical field]

The present invention relates to a method of preparing a heterogeneous linear carbonate adopting catalytic filtering.

### [Background Art]

It is well known that a method of preparing ethyl methyl carbonate (EMC) and diethyl carbonate (DEC), which are used as organic solvents for battery electrolytes is through a transesterification reaction of dimethyl carbonate (DMC) and ethanol.

At this time, a catalyst is used for the reaction, and as the catalyst, sodium methoxide (NaOCH₃, SME) and sodium hydroxide (NaOH), which have excellent activity, are mainly used.

However, since the SME or NaOH has low solubility in organic solvents and is insoluble in DMC, EMC, and DEC, it causes column plugging in a reactive distillation process or a purification process, so that process trouble occurs.

In this regard, in Patent Document 1, EMC and DEC are prepared through a reactive distillation method using DMC, an alcohol, and a catalyst. Referring to drawings, there is a strainer at the rear of a reactive distillation column to separate the solid phase, but when an alcohol is distilled in the reactive distillation column, the catalyst is eventually precipitated, so that powder builds up on the packing inside the column and still causes plugging.

In addition, Patent Document 2 discloses that DEC is produced at a high rate by utilizing the SME catalyst and reactive distillation, wherein the inside of a reactive distillation column is composed of a porous tray, the catalyst is put in a mixed state of SME and an alcohol such as ethanol, but in this method, after the alcohol is distilled inside the reactive distillation column, the catalyst is precipitated in the bottom concentrate, causing plugging, and even if the catalyst passes to the downstream separation process, problems are expected inside an EMC purification tower, and during DEC purification, there is a problem of DEC loss due to precipitated SME.

Therefore, there is a need for research on a method for effectively preparing a heterogeneous linear carbonate for a battery electrolyte by solving the above problems.

### [Related Art Document]

### [Patent Documents]

(Patent Document 1) Chinese Patent Publication No. 103804124
(Patent Document 2) Korean Patent Publication No. 10-1668571

### [Disclosure]

### [Technical Problem]

The present invention is directed to providing a method of preparing a heterogeneous linear carbonate, wherein catalyst filtering is applied to a composite to prepare the heterogeneous linear carbonate, so that a desired compound can be obtained in excellent yield because a catalyst is not precipitated in a purification process.

### [Technical Solution]

One embodiment of the present invention is directed to providing a method of preparing a heterogeneous linear carbonate, including: performing a transesterification reaction of dimethyl carbonate (DMC) and ethanol (EtOH) in the presence of a catalyst; and filtering a composite obtained by the transesterification reaction through a filter.

### [Advantageous Effects]

In a method of preparing a heterogeneous linear carbonate according to the present invention, by applying catalyst filtering to a composite obtained by a transesterification reaction, the low solubility problem of the catalyst is solved, and column plugging due to catalyst precipitation that may occur in a purification process can be prevented, which minimizes process problems and reduces maintenance costs.

In addition, by using a CSTR for the transesterification reaction, it is easy to adjust a production ratio of the desired heterogeneous linear carbonate through adjusting a ratio of dimethyl carbonate and ethanol as raw materials.

In addition, when the ethanol and the catalyst are pre-mixed in a pre-reactor and put into the CSTR, there is an effect of increasing productivity by increasing a reaction rate.

### [Modes of the Invention]

Throughout the specification, when a part is said to "include" a component, this means that the part may further include other components rather than excluding other components unless specifically stated to the contrary.

In the present specification, the unit "% by weight" may mean the ratio of the weight of a certain component to total components.

Hereinafter, the present invention will be described in detail.

One embodiment of the present invention provides a method of preparing a heterogeneous linear carbonate, including:
performing a transesterification reaction of dimethyl carbonate (DMC) and ethanol (EtOH) in the presence of a catalyst; and
filtering a composite obtained by the transesterification reaction through a filter.

In the present invention, the heterogeneous linear carbonate means a species of carbonate which is different from dimethyl carbonate, specifically, an asymmetric linear carbonate and a symmetric linear carbonate, and more specifically, ethyl methyl carbonate (EMC) and diethyl carbonate (DEC).

Catalysts used in the transesterification reaction may generally be one or more selected from the group consisting of lithium methoxide (LME), lithium ethoxide (LEE), sodium methoxide (SME), sodium hydroxide, NaOH) and a mixture thereof, and specifically, may be one or more selected from the group consisting of sodium methoxide (SME) and sodium hydroxide (NaOH) having excellent activity, and a mixture thereof.

However, as described above, these catalysts have low solubility in organic solvents and do not dissolve in dimethyl carbonate as a reaction raw material, ethyl methyl carbonate or diethyl carbonate as a desired product, so that there was a problem of causing column plugging in a reaction process or a purification process.

Accordingly, the inventors of the present application, as a result of in-depth contemplation of how to effectively solve this problem, found that when the composite obtained by the transesterification reaction is filtered, since the catalyst is hardly precipitated in the purification process, it was identified that diethyl carbonate and ethyl methyl carbonate as a desired product, can be produced in excellent yields, and the present invention was completed.

Filtering through the filter is performed by filtering the composite obtained by the transesterification reaction through a filter.

The filter may have pores with an average diameter of 0.3 µm or more and 2 µm or less, specifically, pores with an average diameter of 0.4 µm or more and 1 µm or less, and more specifically, pores with an average diameter of 0.45 µm or more and 0.8 µm or less.

The pores having an average diameter of these filters were measured by magnifying a filter sample surface 2,500 times using a scanning electron microscope (FE-SEM, Hitachi S-4800 Scanning Electron Microscope), and a major axis length among surface pores identified in the randomly sampled range (10 µm or more in width and 15 µm or more in length) in a measured photograph was measured as a pore size. The number of measurements was at least 10, and the average of pore sizes obtained after measurement was obtained.

When an average diameter size is below the above range, it may take a long time to filter, and when the size exceeds the above range, the catalyst may also escape, which is not preferable.

The material of the filter is not limited as long as it has chemical stability and does not react with a carbonate-based material, and for example, may be made of one or more materials selected from the group consisting of polyethylene, polypropylene, and polytetrafluoroethylene (PTFE), specifically, polytetrafluoroethylene (PTFE) material.

In this way, in the case of filtering the composite through the filter having pores, it is possible to prevent catalyst precipitation in a reaction or purification process, and thus the problem of column plugging can be effectively solved.

Meanwhile, such a catalyst may be input in a state of being dissolved in an alcohol-based solvent in an amount of 0.1% by weight or more and 3% by weight or less, specifically 0.1% by weight or more and 2% by weight or less, and more specifically 0.5% by weight or more and 1% by weight or less.

That is, based on the total weight of the solution in which the catalyst is dissolved in the solvent, an amount of the catalyst in the solution is 0.1% by weight or more and 3% by weight or less, specifically 0.1% by weight or more and 2% by weight or less, and more specifically 0.5% by weight or more and 1% by weight or less.

When the wt% of the catalyst exceeds the above range, the content insoluble in the alcohol-based solvent is high and inefficient, and when the wt% is below the above range, a total amount of input solvent increases, and accordingly, energy consumption may increase and reaction efficiency may decrease, which is not preferable.

The alcohol-based solvent may specifically be ethanol.

An amount of these catalysts used may be 0.001% by weight or more and 3% by weight or less, specifically 0.001% by weight or more and 1% by weight or less, and more specifically 0.001% by weight or more and 0.1% by weight or less, based on a weight of dimethyl carbonate.

When the content is below the above range, the reaction does not proceed efficiently, and when the content exceeds the above range, the amount of input catalyst is increased even though an amount of unused catalyst is large, which is not preferable in terms of economics.

Meanwhile, the preparation of a heterogeneous linear carbonate, that is, ethyl methyl carbonate and diethyl carbonate according to the present invention, can be performed in a continuous stirred tank reactor (CSTR), and distillation may be performed in a distillation column. Therefore, the transesterification reaction may be performed by supplying raw materials into the continuous stirred tank reactor (CSTR).

Specifically, in the CSTR, the dimethyl carbonate and ethanol undergo an exchange reaction in the presence of a transesterification catalyst, from which a desired product can be obtained. More specifically, when dimethyl carbonate, ethanol, and the catalyst, which are raw materials for preparation, are continuously supplied to the CSTR, reaction products produced in the reactor are discharged as an effluent stream, and then put into a distillation column to selectively separate and obtain ethyl methyl carbonate and diethyl carbonate as desired products.

At this time, in the present invention, the effluent stream is subjected to the filtering process before being introduced into the distillation column.

In addition, the catalyst and ethanol are supplied in a mixed state in a pre-reactor, and the transesterification reaction may proceed in a continuous stirred tank reactor (CSTR).

That is, the catalyst dissolved in ethanol is put into a pre-reactor together with ethanol as a reaction raw material, mixed in advance, and then the mixture and dimethyl carbonate are each supplied to the CSTR so that the reaction can proceed.

When a pre-mixing process is performed in this way, according to the inventors of the present application, it was confirmed that a faster reaction rate can be obtained. Here, mixing of the catalyst and ethanol is a concept including a reaction by mixing.

Specifically, in the transesterification reaction, since the reaction starts with the OH group of the catalyst attacking the H of ethanol, when the ethanol and the catalyst are first mixed and reacted in advance, the concentration decrease due to dimethyl carbonate can be avoided, which has a beneficial effect on a reaction rate.

Meanwhile, dimethyl carbonate included as a reaction raw material may be purchased and used commercially, and may be those obtained by a gas phase catalytic reaction of carbon monoxide and nitrite ester, those obtained by reacting carbon dioxide and an alcohol under a solid catalyst, and dimethyl carbonate produced by a known method.

As another reaction raw material, commercially available ethanol may be used as it is, but it is preferable to use ethanol having a moisture content of 0.20% by mass or less (2000 ppm or less) so as not to affect the transesterification reaction of the present invention. Here, the removal of the contained water is performed by dehydration operation or the like with a drying agent such as, for example, a molecular sieve, anhydrous magnesium sulfate and/or calcium oxide.

An amount of ethanol used may be 20% by weight or more and 150% by weight or less, specifically, 30% by weight or more and 130% by weight or less, more preferably 40% by weight or more and 130% by weight or less, based on the weight of dimethyl carbonate.

When the input amount of ethanol is too small, the reaction does not proceed efficiently, meanwhile, when the input amount is too much, the complexity of removing the ethanol after the reaction increases, and it is also not preferable in terms of economics.

Meanwhile, a reaction temperature of the transesterification reaction is affected by the temperature in the CSTR, which is a reactor. The reaction temperature may be 30 °C or more and 130 °C or less, specifically, 60 °C or more and 120 °C or less, more specifically 80 °C or more and 100 °C or less, and therefore, the temperature in the reactor may also be adjusted to this temperature range.

When the reaction temperature is below the above range, the reaction is not easily performed and the reaction efficiency is low, and when the reaction temperature exceeds the above range, energy costs increase and an amount of reaction by-products increases, which are not preferable.

In addition, the pressure of the reaction is not particularly limited, and may vary depending on the reaction temperature and reaction composition, and may be, for example, normal pressure to 1000 kPa.

In addition, the pH of the reaction is maintained in the range of 6 or more and 9 or less. An alkali metal compound or alkaline earth metal compound may be added to the reaction when necessary to maintain the pH. Exemplary alkaline earth metal compounds include, but are not limited to, oxides, hydroxides, carbonates, and carboxylic acid salts.

The reaction time varies depending on reaction conditions, reaction raw materials, and other factors that may affect the reaction. Typically, however, the reaction time is between 0.5 and 20 hours. In the case of a continuous process using a CSTR, the reaction time (retention time) is determined by the kinetics of a system, which is determined by the used pressure, temperature and catalyst.

Thus, conditions of a transesterification reaction by a CSTR includes the reaction pressure, temperature, concentrations of the reactants, pH, and reaction time suitable to produce a desired reaction product. Any specific conditions used in this process are not particularly limited and are selected based on the reaction raw materials and composites produced using the process.

In this way, when the transesterification reaction is completed using the CSTR, the reaction concentrate and the composite including ethyl methyl carbonate and diethyl carbonate are obtained as an effluent stream. In addition, when this is filtered through a filter and then subjected to distillation, high purity ethyl methyl carbonate and diethyl carbonate can be obtained in excellent yield without catalyst precipitation.

In addition, according to the present invention, when the catalyst is mixed and reacted with ethanol in advance, a reaction rate can be increased to avoid the concentration decrease due to DMC, so that the economic efficiency is very excellent.

Hereinafter, examples will be given to describe the present invention in detail. However, the examples according to the present invention may be modified into various other forms, and the scope of the present invention is not to be construed as being limited to the examples described below. The examples of the present specification are provided to more completely explain the present invention to those of ordinary skill in the art.

### [Example 1]

540.48 g of dimethyl carbonate (DMC), 360.592 g of ethanol (EtOH), and 54.048 g of a sodium methoxide solution (SME, 1 wt% in ethanol (EtOH)) were used as raw materials, and put into a CSTR having a volume of 100 ml at a rate of 1.67 ml/min to synthesize ethyl methyl carbonate and diethyl carbonate. At this time, the reaction was performed at 70 °C, 1 bar, 3 hours, and pH 12.

After the reaction, 300 g of a composite in an effluent stream was passed through a filter having a pore diameter of 0.45 µm and then distilled.

### [Example 2]

1080.96 g of dimethyl carbonate (DMC), 721.184 g of ethanol (EtOH), and 108.096 g of a sodium methoxide solution (SME, 1 wt% in ethanol (EtOH)) were used as raw materials, and put into a CSTR having a volume of 100 ml at a rate of 10 g/min to synthesize ethyl methyl carbonate and diethyl carbonate. At this time, the ethanol and the SME were pre-mixed in a pre-reactor, and when put into the CSTR, a mixture of the ethanol and SME pre-mixed with the DMC was input. At this time, the reaction was performed at 70 °C, 1 bar, 1 hour, and pH 12.

After the reaction, 300 g of a composite in an effluent stream was passed through a filter having a pore diameter of 0.45 µm and then distilled.

### [Comparative Example 1]

Ethyl methyl carbonate and diethyl carbonate were synthesized in the same manner as in Example 1, except that a batch reactor was used instead of the CSTR and reaction conditions were adjusted to 70 °C, 1 bar, 1 hour, and pH 12.

Furthermore, 300 g of the composite obtained by the reaction was distilled.

### [Comparative Example 2]

Ethyl methyl carbonate and diethyl carbonate were synthesized in the same manner as in Comparative Example 1.

Further, 300 g of a composite was distilled from the effluent stream after the reaction.

### [Experimental Example 1]

Qualitative and quantitative analyses were performed on the consumption amount of dimethyl carbonate as a raw material for preparation and production amounts of ethyl methyl carbonate and diethyl carbonate as desired products in Examples 1 and 2 and Comparative Examples 1 and 2, and results are shown in Table 1 below.

For the qualitative and quantitative analyses, 1 g of an obtained product was taken and mixed with 0.1 g of m-xylene, and then the concentration was measured using gas chromatography (GC) (YL6500GC manufactured by YOUNG IN Chromass Co., GC column: DB-1 30 m×0.53 mm, GC detector: FID). In addition, a reaction conversion rate of dimethyl carbonate as a raw material for preparation was calculated as mol% based on a consumption amount compared to an amount used, and the reaction selectivity of ethyl methyl carbonate and diethyl carbonate as desired products was calculated as mol% based on a total content of ethyl methyl carbonate and diethyl carbonate respectively produced.

In addition, after the distillation was completed, a solid catalyst in the residue was weighed, and the results are also shown in Table 1 below.

**[Table 1]**

| Classification | | Example 1 | Example 2 | Comparative Example 1 | Comparative Example 2 |
|---|---|---|---|---|---|
| Activity results | DMC conversion rate [%] | 65 | 42 | 65 | 35 |
| | EMC selectivity [%] | 74 | 89 | 73 | 91 |
| | DEC selectivity [%] | 26 | 11 | 27 | 9 |
| Amount of solid catalyst in residue [g] | | 0.015 | 0.012 | 0.165 | 0.164 |

According to Table 1, when reacting in a CSTR and adopting a filtering process, which is the method according to the present invention, it can be confirmed that activity of the catalyst is the same as in the case of performing the conventional method, but a precipitation amount of the catalyst is significantly reduced by a filtering process and there is almost no precipitation amount.

In addition, referring to Example 2 and Comparative Example 2, when the catalyst is reacted with ethanol in advance and put into the CSTR, it can be confirmed that a DMC conversion rate is high at the same reaction time and a reaction rate is increased.

## Claims

1. A method of preparing a heterogeneous linear carbonate, comprising:
performing a transesterification reaction of dimethyl carbonate (DMC) and ethanol (EtOH) in the presence of a catalyst; and
filtering a composite obtained by the transesterification reaction through a filter.

2. The method of claim 1, wherein the filter has pores having an average diameter of 0.3 µm or more and 2 µm or less.

3. The method of claim 1, wherein the filter is made of one or more materials selected from the group consisting of polyethylene, polypropylene, and polytetrafluoroethylene (PTFE).

4. The method of claim 1, wherein the catalyst is input in a state of being dissolved in an alcohol-based solvent in an amount of 0.1% by weight or more and 3% by weight or less.

5. The method of claim 1, wherein the catalyst is added in an amount of 0.001% by weight or more and 3% by weight or less based on a weight of dimethyl carbonate.

6. The method of claim 1, wherein the catalyst and the ethanol are supplied in a mixed state in a pre-reactor, and the transesterification reaction is performed in a continuous stirred tank reactor (CSTR).

7. The method of claim 1, wherein the catalyst is one or more selected from the group consisting of sodium methoxide (SME), sodium hydroxide (NaOH), and a mixture thereof.
